# EUROPEAN PATENT SPECIFICATION

(11) **EP 2 333 056 B1**
(45) Date of publication and mention of the grant of the patent: **07.05.2014**
(21) Application number: 11156545.3
(22) Date of filing: 12.04.2007
(51) Int. Cl.: C12N 9/96, A23C 19/032, C12N 9/52, C12N 9/58, C12N 9/64

(54) **Liquid composition comprising an aspartic protease**
Flüssige Mischung die eine Aspartat-protease enthält
Composition liquide comprenant une protéase aspartique

(30) Priority: 13.04.2006 EP 06112649; 13.04.2006 US 791447 P
(43) Date of publication of application: 15.06.2011
(62) Divisional of application: 07728023.8
(73) Proprietor: DSM IP Assets B.V., 6411 TE Heerlen (NL)
(72) Inventor: Haan, De, André, 2645 GC, DELFGAUW (NL); Caussette, Mylene, 59000, LILLE (FR); Schooneveld-Bergmans, Margot, Elisabeth, Francoise, 2625 KK, DELFT (NL)
(74) Representative: van Grieken-Plooster, Izabella Johanna

(56) References cited:
- WO-A-95/29999
- US-A- 3 763 010
- M.L. CABO, A.F. BRABER, P.M.F.J. KOENRAAD: "Apparent Antifungal Activity of Several Lactic Acid Bacteria against Penicillium discolor is Due to Acetic Acid in the Medium", JOURNAL OF FOOD PROTECTION, DES MOINES, IO, US, vol. 65, no. 8, 2002, pages 1309-1316, XP008068821, ISSN: 0362-028X

## Description

### Description of the invention

The present invention relates to a liquid composition comprising an aspartic protease.

It is well known that the preparation of cheese involves the use of an aspartic protease. The aspartic protease causes the milk to coagulate, resulting in a solid curd which is further processed into cheese.

Aspartic proteases can be recovered from animals, e.g. from the stomach of calf, camel, and seal. They can also be produced by micro-organisms, for instance *Rhizomucor, Cryphonectria,* or host strains such as *Aspergillus or Kluyveromyces.*

In the cheese making industry, liquid compositions comprising the aspartic protease are often used. Such liquid compositions typically contain certain additives to obtain a desired stability. One can distinguish between enzymatic stability and microbial stability. The enzymatic stability is a measure for the rate at which the activity of the enzyme decreases. The microbial stability is a measure for the rate at which micro-organisms can proliferate and grow in the composition.

The microbial properties of a composition can be expressed by the standard plate count, number of yeasts and number of moulds using well-defined standard procedures. For instance, the standard plate count can be ≤ 100 in 1 ml, the yeast count can be ≤ 10 in 1 ml and the mould count can be ≤ 10 in 1 ml. As compositions are often stored prior to use, it is desirable that the plate count, number of yeasts and number of moulds remain below certain boundary values, for instance the values mentioned above, for a prolonged period, for instance for a period of at least 3 months.

It is well known to use sorbate or benzoate as a preservative to obtain a desired microbial stability. Parabens (alkyl esters of para-hydroxybenzoate) can also be used.

For instance, US-A-3763010 discloses a composition comprising an aspartic protease, and potassium sorbate and sodium benzoate. WO-A-9015865 and WO-A-9529999 disclose that sodium benzoate is used in compositions containing an aspartic protease. Commercial compositions comprising an aspartic protease and between 3 and 5 g/l (between 0.02 and 0.035 mol/l) of sodium benzoate are known.

However, there is a desire for products containing no or smaller amounts of sorbates, benzoates and parabens.

The goal of the invention is to provide a composition which has a good microbial stability in the absence of sorbates, benzoates and parabens.

US-A-3 763 010 describes a stabilized microbial rennet from Mucor miehei by admixing a solid enzyme product recovered from fermentation broth with 2-3 percent of gatty acid monoesters of polyoxyethylene sorbitan.

WO 95/29999 describes a process for separating milk clotting enzymes from extracts of animal stomach tissue. WO 95/29999 further describes :a liquid chymosin composition a chymosin stabilizing agent.

Cabo et al. (Journal of food protection des Moines, IO, US, vol. 65, no. 8, 2002, pages 1309-1316) describes that the antifungal activity of several lactic acid bacteria against Penicillium discolor is due to acetic acid in the used medium.

The invention provides compositions which can have a surprisingly high microbial stability, even in the absence of these compounds or in the presence of these compounds in quantities lower than known in the prior art. In addition, the compositions can have a surprisingly high enzymatic stability. The compositions according to the invention can have a longer shelf life than expected.

According to a first aspect of the invention, there is provided a liquid composition comprising:
(i) a *Rhizomucor miehei* aspartic protease; and
(ii) an inorganic salt; and
(iii) a compound selected from formate, acetate, lactate, propionate, malate or fumarate in which composition;
the sum concentration of sorbate, benzoate and alkyl esters of para-hydroxybenzoate is less than 0.010 mol/l;
the standard plate count ≤ 100 in 1 ml;
yeast count ≤ 10 in 1 ml; and
mould count ≤ 10 in 1 ml,
and wherein the pH is between 4.8 and 5.5; and
in which the concentration of formate, acetate, lactate, propionate, malate, or fumarate or combination thereof is at least 0.1 mol/l.

It will be understood that sorbic acid as well as salts of sorbic acid contribute to the concentration of sorbate. Benzoic acid as well as salts of benzoic acid contribute to the concentration of benzoate. Alkyl esters of para-hydroxybenzoate may or may not be in the form of a salt. Accordingly, as used herein, the sum concentration of sorbate, benzoate and alkyl esters of para-hydroxybenzoate refers to the sum concentration of sorbic acid, salts of sorbic acid, benzoic acid, salts of benzoic acid, alkyl esters of para-hydroxybenzoate and salts of alkyl esters of para-hydroxybenzoate in the composition. Examples of salts of sorbic acid are sodium sorbate, potassium sorbate, and calcium sorbate. Examples of salts of benzoic acid are sodium benzoate, potassium benzoate and calcium benzoate. Examples of alkyl esters of para-hydroxybenzoates are methyl-p-hydroxybenzoate, ethyl-p-hydroxybenzoate and propyl-p-hydroxybenzoate. Examples of salts of alkyl esters of para-hydroxybenzoates are sodium salt of methyl-p-hydroxybenzoate, the sodium salt of ethyl-p-hydroxybenzoate, and the sodium salt of propyl-p-hydroxybenzoate.

As used herein, the standard plate count is determined according to ISO 4833:1991 (E) (Microbiology - General guidance for the enumeration of micro-organisms - Colony count technique at 30°C).

The yeast count is determined according to ISO 7954: 1987 (E) (Microbiology - General guidance for enumeration of yeasts and moulds - Colony count technique at 25°C).

The moulds count is determined according to ISO 7954: 1987 (E) (Microbiology - General guidance for enumeration of yeasts and moulds - Colony count technique at 25°C).

The invention provides a liquid composition comprising an aspartic protease and a compound selected from formate, acetate, lactate, propionate, malate, or fumarate. These compounds can contribute to the microbial stability. It will be understood that these compounds are the anions of the corresponding organic acids (formic acid, acetic acid, lactic acid, propionic acid malic acid and fumaric acid), and that these compounds may be supplemented to the composition as the organic acid or the salt thereof. The salt may for instance be a potassium salt, a sodium salt or a calcium salt.

The organic acid and/or salt thereof may be employed in any suitable concentration. In a preferred embodiment, the concentration of formate, acetate, lactate, propionate, malate, or fumarate or combination thereof in the composition is at least 0.1 mol/I, for instance at least 0.2 mol/l. It will be understood that it is possible that at least one of these compounds is present in a preferred concentration as defined herein. It is also possible that a combination of two or more of these compounds is present in a preferred concentration as defined herein. If a combination is employed, the concentration refers to the sum concentration of these compounds. There is no specific upper limit for the concentration. The concentration of formate, acetate, lactate, propionate, malate, or fumarate or combination thereof may be less than 2 mol/l, for instance less than 1 mol/l.

In a preferred embodiment, the composition comprises acetate. Preferably, the composition comprises at least 0.1 mol/l, for instance at least 0.2 mol/l of acetate. There is no specific upper limit for the concentration of acetate. The composition may for instance comprise less than 2 mol/l, for instance less 1 mol/l of acetate.

According to an aspect of the invention, there is provided a liquid composition comprising:
(i) a *Rhizomucor miehei* aspartic protease; and
(ii) an inorganic salt; and
(iii) a compound selected from formate, acetate, lactate, propionate, malate or fumarate in which composition;
the sum concentration of sorbate, benzoate and alkyl esters of para-hydroxybenzoate is less than 0.010 mol/l;
the standard plate count ≤ 100 in 1 ml;
yeast count ≤ 10 in 1 ml; and
mould count ≤ 10 in 1 ml,
and wherein the pH is between 4.8 and 5.5; and
in which the concentration of formate, acetate, lactate, propionate, malate, or fumarate or combination thereof is at least 0.1 mol/l., in which composition the concentration of inorganic salt is less than 180 g/l, preferably less than 170 g/l, more preferably less than 160 g/l. It will be understood that the composition may contain one or more inorganic salts. The values for the preferred upper limits of the concentration of inorganic salt refer to the sum concentration of the inorganic salts in the composition.

According to an aspect of the invention, there is provided a process for preparing a liquid composition comprising a *Rhizomucor miehei* aspartic protease, said process comprising:
(a) providing a fermentation broth, said fermentation broth containing (i) micro-organisms that have produced the protease and (ii) supernatant containing the protease;
(b) separating, by solid liquid separation, supernatant from the fermentation broth;
(c) purifying the separated supernatant, to obtain a purified solution;
(d) optionally, adding one or more additives to the purified solution, wherein at least one of said one or more additives is an inorganic salt, a polyalcohol, or a compound selected from formate, acetate, lactate, propionate, malate, or fumarate; and
(e) filtering the purified solution, containing said one or more additives, and wherein the concentration of formate, acetate, lactate, propionate, malate, or fumarate or combination thereof is at least 0.1 mol/l.

Also described is a compostion obtainable by the described process.

Preferred embodiments will be described hereinafter, and are applicable to the invention in all its aspects. It will further be appreciated that the present invention further includes any combination of the various aspects of the invention and/or preferred features.

According to the invention the use of benzoate, sorbate or para-hydroxybenzoate is not necessary or benzoate, sorbate or para-hydroxybenzoate can be used in smaller quantities. In a preferred embodiment, the composition according to the invention comprises less than 0.010 mol/l of benzoate, preferably less than 0.005 mol/l, preferably less than 0.002 mol/l, preferably less than 0.001 mol/l, preferably less than 0.0005 mol/l, preferably less than 0.0001 mol/l, preferably less than 0.00005 mol/l, preferably less than 0.00001 mol/l, preferably no detectable amount. In a further preferred embodiment, the sum concentration of sorbate, benzoate and alkyl esters of para-hydroxybenzoates in the composition according to the invention is less than 0.010 mol/l, preferably less than 0.005 mol/l, preferably less than 0.002 mol/l, preferably less than 0.001 mol/l, preferably less than 0.0005 mol/l, preferably less than 0.0001 mol/l, preferably less than 0.00005 mol/l, preferably less than 0.00001 mol/l, preferably no detectable amount. It is known that benzoate, sorbate and parahydroxybenzoate may be used to kill the micro-organisms after a fermentation. Accordingly, small amounts of these compounds may be present in the composition resulting from such killing step.

In another embodiment of the invention, the sum concentration of preservatives in the composition according to the invention is less than 0.010 mol/l, preferably less than 0.005 mol/l, preferably less than 0.002 mol/l, preferably less than 0.001 mol/l, preferably less than 0.0005 mol/l, preferably less than 0.0001 mol/l, preferably less than 0.00005 mol/l, preferably less than 0.00001 mol/l, preferably no detectable amount. The term preservative is well understood by the person skilled in the art, and, as used herein, refers to a compound which inhibits the growth of microorganisms and/or prevents the germination of spores and/or kills vegetative cells and/or spores. In the context of the present invention, this definition of preservative does not encompass polyalcohols or inorganic salts.

Preferably, the liquid composition is an aqueous composition, for instance an aqueous solution. As used herein an aqueous composition or aqueous solution encompasses any composition or solution comprising water, for instance at least 20 wt.% of water, for instance at least 40 wt.% of water.

The composition according to the invention has a water activity of below 0.95, for instance below 0.92, for instance below 0.9, for instance below 0.85, for instance below 0.8. As used herein the water activity refers to the value measured at 25 °C. A relatively low water activity can contribute to achieve a desired microbial stability. The water activity can be influenced by the addition of inorganic salt and/or polyalcohols. The water activity may be above 0.7. for instance above 0.8, for instance above 0.83, or above 0.85 or above 0.86. It was surprisingly found that a composition having a water activity above these values can have a higher microbial stability than expected. The water activity is preferably between 0.85 and 0.95.

The composition according to the invention comprises an inorganic salt. The inorganic salt can function to decrease the water activity of the composition. Any suitable inorganic salt may be used. The inorganic salt may for instance be a salt which comprises a cation selected from the group consisting of (CH₃)₄N⁺, NH4⁺, K⁺, Na⁺, Ca²⁺ and Ba²⁺ and an anion selected from the group consisting of SO₄²⁻, Cl⁻, Br⁻, NO₃⁻, ClO₄⁻ and SCN-. Preferred inorganic salts are NaCl, KCI, Na₂SO₄ or (NH₄)₂SO₄. The composition may contain one or more inorganic salts.

In a preferred embodiment, the composition comprises at least 80 g/I of an inorganic salt or of a combination of inorganic salts, preferably at least 100 g/I, more preferably at least 120 g/I, more preferably at least 140 g/I. An increased salt concentration has the effect that the water activity of the composition is increased, which can assist to achieve a desired microbial stability. It will be understood that it is possible that at least one inorganic salt is present in a preferred concentration as defined herein. It is also possible that a combination of inorganic salts is present in a concentration as defined herein. If a combination is of inorganic salts is employed, the concentration refers to the sum concentration of inorganic salts. In a preferred embodiment, the composition comprises at least 80 g/I of NaCl, preferably at least 100 g/I, more preferably at least 120 g/l, more preferably at least 140 g/I.

In a preferred embodiment, the concentration of inorganic salt in the composition is less than 200 g/l, preferably less than 190 g/I, preferably less than 180 g/l, preferably less than 170 g/I, for instance less than 160 g/I. In a preferred embodiment, the composition comprises less than 200 g/I, preferably less than 190 g/I, preferably less than 180 g/I, preferably less than 170 g/I, for instance less than 160 g/l of NaCl. Decreasing the concentration of inorganic salt can contribute to the enzymatic stability. The invention enables the use of relatively low concentrations of inorganic salts, which can assist in achieving a combination of a high microbial and a high enzymatic stability. It will be understood that the composition may contain one or more inorganic salts. The values for the preferred upper limits of the concentration of inorganic salt refer to the sum concentration of the inorganic salts in the composition.

The composition can comprises a polyalcohol. The polyalcohol can function to decrease the water activity of the composition. Decreasing the water activity can assist in achieving a desired microbial stability. Any suitable polyalcohol may be used. The polyalcohol may for instance be ethylene glycol (ethanediol), propylene glycol (propanediol), glycerol, erythritol, xylitol, mannitol, sorbitol, inositol, galactitol. Preferably, the polyalcohol is glycerol, sorbitol or propanediol, more preferably glycerol or propanediol. The composition may comprise one or more polyalcohols.

The composition may comprises at least 40 g/I of a polyalcohol or of a combination of polyalcohols, preferably at least 80 g/I. It will be understood that it is possible that at least one polyalcohol is present in a preferred concentration as defined herein. It is also possible that a combination of polyalcohols is present in a concentration as defined herein. If a combination is of polyalcohols is employed, the concentration refers to the sum concentration of inorganic salts.

The concentration of polyalcohol in the composition may be less than 300 g/I, for instance less than 200 g/I, for instance less than 150 g/l. However, lower concentrations, for instance concentrations less than 100 g/l, for instance less than 50 g/l, for instance less than 10 g/l or even 0 g/I may also be used. The upper limits refer to the sum concentration of polyalcohols in the composition.

The composition may comprises between 90 and 120 g/I of a polyalcohol or of a combination of polyalcohols, and, preferably, between 140 and 180 g/l of NaCl.

The pH is between 4.8 and 5.5.

In a preferred embodiment, the composition comprises a reducing agent, preferably methionine. Preferably, the composition comprises at least 1 g/l of methionine, preferably at least 2 g/I, more preferably at least 5 g/l, for instance less than 100 g/I, for instance less than 30 g/I.

The enzyme activity is at least 100 IMCU per ml of composition, preferably at least 200 IMCU per ml of composition, preferably at least 500 IMCU per ml of composition. There is no specific upper limit for the enzyme activity. The enzyme activity may be below 5000 IMCU per ml of composition, for instance less than 2000 IMCU per ml, for instance less than 1000 lMCU per ml of composition. IMCU refers to International Milk Clotting Unit, defined by the International Dairy Federation (IDF), protocol 176: 1996.

The composition according to the invention has standard plate count ≤ 100 in 1 ml. Preferably, the yeast count ≤ 10 in 1 ml. Preferably, the mould count is ≤ 10 in 1 ml.

The composition according to the invention has a higher microbial stability than expected. In an embodiment of the invention, the standard plate count remains ≤ 100 in 1 ml, the yeast count remains ≤ 10 in 1 ml and the mould count remains ≤ 10 in 1 ml during a period of at least 4 months, preferably at least 6 months, preferably at least 9 months, preferably at least 12 months, preferably at least 18 months, preferably at least 24 months, when the composition is stored in a closed container at a temperature of 4 °C in the dark.

In an embodiment of the invention, the standard plate count remains ≤ 100 in 1 ml, the yeast count remains ≤ 10 in 1 ml and the mould count remains ≤ 10 in 1 ml during a period of at least 4 months, preferably at least 6 months, preferably at least 9 months, preferably at least 12 months, preferably at least 18 months, preferably at least 24 months, when the composition is stored in a closed container at a temperature of 30 °C in the dark.

In an embodiment of the invention, the enzymatic activity decreases at most 5% during a period of at least 4 months, preferably at least 6 months, preferably at least 9 months, preferably at least 12 months, preferably at least 18 months, preferably at least 24 months, when the composition is stored in a closed container at a temperature of 4 °C in the dark.

In an embodiment of the invention, the enzymatic activity decreases at most 5% during a period of at least 4 months, preferably at least 6 months, preferably at least 9 months, preferably at least 12 months, preferably at least 18 months, preferably at least 24 months, when the composition is stored in a closed container at a temperature of 30 °C in the dark.

As closed container for these tests can be used a bottle, which is sterilized prior to filling, and that is closed with a screw thread. Bottles having a volume of 50 ml may be used which are filled with 20 ml of the composition to be tested.

The composition comprises an aspartic protease. Preferably, the aspartic protease is a milk clotting enzyme. Milk clotting enzymes can be characterized by having specificity for the peptide bond between residue 105 phenylalanine and residue 106 methionine or a bond adjacent to that in kappa-casein.

The aspartic protease may be of animal origin. Preferably, the aspartic protease is produced by a micro-organism (a microbially produced aspartic protease).

The microorganism may for instance be *Rhizomucor,* for instance *Rhizomucor miehei* or *Rhizomucor pussilus* or *Cryphonectria,* for instance *Cryphonectria parasitica.* The microorganism may also be selected from the genera of *Aspergillus, Trichoderma, Penicillium, Fusarium, Humicola,* or *Kluyveromyces.* These microorganisms may for instance be used as host strain. In a preferred embodiment the microorganism is *Aspergillus niger, Aspergillus nidulans, Aspergillus oryzae, Kluyveromyces lactis,* or *Escherichia coli.*

In the claimed composition, the aspartic protease is a *Rhizomucor miehei* aspartic protease. The term *"Rhizomucor miehei* aspartic protease" encompasses the aspartic protease homologously produced in *Rhizomucor miehei.* A process for the preparation of the enzyme via fermentation is described in US-A-3,988,207. The term *"Rhizomucor miehei* aspartic protease" also encompasses a recombinant *Rhizomucor miehei* aspartic protease, for example a *Rhizomucor miehei* aspartic protease produced in a host organism (e.g. other than *Rhizomucor miehei)* transformed with DNA coding for the *Rhizomucor miehei* aspartic protease. A method for the production of a recombinant *Rhizomucor miehei* aspartic protease in a host organism is described in EP-A-700253.

Another aspartic protease is chymosin. Chymosin may for instance be extracted from the stomach of a calf, camel or seal. In a preferred embodiment of the invention the chymosin is produced by a microorgansim, e.g. via recombinant DNA technology in bacteria, e.g. *Escherichia coli,* yeast, e.g. *Kluyveromyces lactis,* or filamentous fungi, e.g. in *Aspergillus niger.*

The composition according to the invention can be packaged in any suitable closed container. Accordingly, the invention further provides a closed and/or sealed container containing the composition according to the invention.

The composition according to the invention can be prepared using a process for preparing a liquid composition comprising a *Rhizomucor miehei* aspartic protease, said process comprising:
(a) providing a fermentation broth, said fermentation broth containing (i) micro-organisms that have produced the protease and (ii) supernatant containing the protease;
(b) separating, by solid liquid separation, supernatant from the fermentation broth;
(c) purifying the separated supernatant, to obtain a purified solution;
(d) optionally, adding one or more additives to the purified solution, wherein at least one of said one or more additives is an inorganic salt, a polyalcohol, or a compound selected from formate, acetate, lactate, propionate, malate, or fumarate; and
(e) filtering the purified solution, containing said one or more additives, and wherein the concentration of formate, acetate, lactate, propionate, malate, or fumarate or combination thereof is at least 0.1 mol/l.

Step (a) can be carried out in any suitable manner and may involve culturing a micro-organism under conditions suitable to produce the protease, resulting in a fermentation broth containing the micro-organism and supernatant containing the protease. A suitable process is for instance described in EP-A-1365019.

Step (b) can be carried out in any suitable manner, and preferably involves centrifugation and/or filtration. Step (b) may involve filtering using a membrane filter press or a polish filter.

Step (c) functions may involve any step which results in an increase of the concentration of the enzyme relative to the other components. Preferably, step (c) involves chromatography or ultrafiltration. Preferred processes for performing chromatography are described in WO-A-03100048 and WO-A-0250253.

Step (d) involves adding one or more additives to the purified solution, wherein at least one of said one or more additives is an inorganic salt, a polyalcohol, or a compound selected from formate, acetate, lactate, propionate, malate, or fumarate. Other additives may also be added. Preferably, no compound selected from benzoate, sorbate or alkyl ester of para-hydroxybenzoate is added to the purified solution.

Step (e) preferably involves polish filtration or sterile filtration. Polish filtration is well known per se. Polish filtration in step (e) functions to remove trace amounts of non dissolved particles, for instance cell debris, and/or contaminating micro-organisms. Polishing filters typically have a relatively small radii of the filter pores (micrometer range) and a shallow depth of the active filter layer (millimeter to centimeter range).

Preferably, the filtered solution resulting from (e) has the following properties: standard plate count ≤ 100 in 1 ml; yeast count ≤ 10 in 1 ml; and moulds count ≤ 10 in 1 ml.

Preferably, equipment that is contacted with the filtered solution resulting from (e) is contacted with steam prior to contacting the equipment with the filtered solution. This avoids contamination,

One or more of steps (a), (b), (c), (d) or (e) may be carried out at a temperature less 10 °C, preferably less than 5 °C.

In a preferred embodiment, the sum concentration of sorbate, benzoate and alkyl esters of para-hydroxybenzoate in the filtered solution resulting from (e) is less than 0.010 mol/l, preferably less than 0.005 mol/l, preferably less than 0.002 mol/l, preferably less than 0.001 mol/l, preferably less than 0.0005 mol/l, preferably less than 0.0001 mol/l, preferably less than 0.00005 mol/l, preferably less than 0.00001 mol/l, preferably no detectable amount.

The invention further provides the use composition according to the invention as a coagulant in the production of cheese.

The invention further provides a process for preparing cheese, comprising, (i) supplementing milk with a composition according to the invention, to effect coagulation of the milk, wherein a curd is obtained; and (ii) processing the curd into cheese.

The invention will now be elucidated with reference to the following examples, without, however, being limited thereto.

### EXAMPLES

### Examples 1-5

A culture of *Rhizomucor miehei was* cultured as described in EP-A-1365019. At the end of fermentation the broth was cooled, the fungus killed off and separated from the liquid using a membrane filter press and polish filtration. The milk clotting protease was subsequently purified using chromatography as described in WO03/100048. The column eluate, containing the milk clotting protease was formulated by adding NaCl, Sodium Acetate, Methionine (10 g/l), and optionally sodium benzoate, and by adjustment of the pH (see table 1). Conditions were specifically selected such that contamination was avoided which included steaming of vats and piping.

**Table 1. Formulations of milk clotting protease from Rhizomucor miehei.**

| exm | pH | NaCl (g/l) | Sodium Acetate (g/l) | Water activity | Initial enzyme activity (IMCU) | Sodium Benzoate (g/l) |
|---|---|---|---|---|---|---|
| 1 | 5.0 | 150 | 30 | 0.88 | 616 | 4.5 |
| 2 | 5.0 | 150 | 30 | 0.88 | 624 | - |
| 3 | 5.3 | 165 | 30 | 0.86 | 688 | - |
| 4 | 4.8 | 165 | 30 | 0.86 | 714 | - |
| 5 | 4.2 | 165 | 30 | 0.86 | 724 | - |

The water activity was determined using a Thermoconstanter TH-200 (Novasina, Axair Ltd, Switzerland) at 25°C, and calibrated with 6 calibration salts of 11, 33, 53, 75, 90 and 98% relative humidity, as supplied by the manufacturer.

### Stability test

Samples (20 ml) were stored in an incubator preset at 30°C in bottles closed with a screw top. At various time intervals (0, 1, 2, 4 weeks, 2 , 8 months) standard plate count was determined, according to ISO 4833:1991 (E) (Microbiology - General guidance for the enumeration of micro-organisms - Colony count technique at 30°C), as well as yeast count and mould count, according to ISO 7954: 1987 (E) (Microbiology - General guidance for enumeration of yeasts and moulds - Colony count technique at 25°C). In addition to these determinations - which were performed using a culture medium without NaCl - determinations were also performed in the same manner, but with the difference that a culture medium with 5% NaCl was used.

In all samples colony counts were < 10 per ml for bacteria, yeasts and moulds, whether cell counts were performed on plates without or with NaCl.

This stability test shows that in all formulations without benzoate the standard plate count remains ≤ 100 in 1 ml; yeasts remains ≤ 10 in 1 ml; and moulds remains≤ 10 in 1 ml, when a sample is stored at 30 °C during a period of at least 8 months. The standard plate count remains ≤ 100 in 1 ml; yeasts remains ≤ 10 in 1 ml; and moulds remains ≤ 10 in 1 ml, when a sample is stored at 4 °C during a period of at least 12 months.

### Challenge test

The 5 formulations of the milk clotting protease from *Rhizomucor miehei* were subjected to challenge tests, in which selected microorganisms of xerotolerant bacteria, yeasts and moulds were inoculated. It was found that all formulations have a good resistance to microorganisms.

During this period of storage the enzymatic activity of the milk clotting protease, as determined in IMCU (International Milk Clotting Unit, defined by the International Dairy Federation (IDF), protocol 176: 1996) decreased less than 0.5% per month in formulations 1, 2 and 3. In the formulations 4 and 5 the decrease was larger.

### Examples 6-9

*A Rhizomucor miehei* aspartic protease was produced as described in Examples 1-5. The product was formulated as given in Table 2. Initial enzyme activity was 760 IMCU/ml.

**Table 2. Formulations of milk clotting protease from Rhizomucor miehei.**

| Ex | pH | NaCl (g/l) | Sodium acetate (g/l) | Sodium benzoate (g/l) |
|---|---|---|---|---|
| 6 | 5.4 | 140 | 30 | 4.5 |
| 7 | 5.4 | 140 | 30 | - |
| 8 | 5.4 | 140 | - | - |
| 9 | 5.1 | 200 | - | - |

### Stability test

Samples (50 ml) were stored in an incubator set at 20°C in bottles closed with a screw top. At various time intervals (0, 1, 2, 4, and from then on every 4 weeks up to 8 months) standard plate count was determined as described in Examples 1-5. In addition to these determinations - which were performed using a culture medium without NaCl - determinations were also performed in the same manner, but with the difference that a culture medium with 10% NaCl was used. The standard plate count without 10% NaCl in the medium showed < 10 colony forming units per ml from the start through the entire storage period, whereas for the formulations 8 and 9 without acetate and benzoate this level was reached after 2 weeks of storage, and then stayed constant for the whole storage period. The standard plate count with 10% NaCl in the medium showed for all formulations from the start until the end of the storage period < 10 colony forming units per ml.

This stability test shows that acetate gives good microbial stability, even in the absence of benzoate.

### Challenge tests

The 4 formulations of the aspartic protease of *Rhizomucor miehei,* as given in Table 2, were inoculated with a mix of xerotolerant microorganisms (a halophilic *Micrococcus, Torulopsis candida* and *Hansenula anomala)* at approximately 2E+3 colony forming units per ml. Samples were stored in an incubator set at 20°C, in bottles closed with a screw top. It was found that the formulations of examples 6 & 7 (containing benzoate or acetate) have an improved resistance to microorganisms compared to example 8 which does not contain benzoate or acetate. Although the formulation of example 9 was found to have a good resistance against microorganisms, the use of acetate was found to enable the use of lower salt concentrations which can improve the enzymatic stability.

Overall, both the stability and challenge test demonstrate the efficient preservative effect of acetate, even in the absence of benzoate.

### Examples 10-11

*A Rhizomucor miehei* aspartic protease was produced as described in Examples 1-5. The product was formulated as given in Table 3, including 10 g/l of methionine. After pH correction, and addition of the various compounds, the formulated product was again subjected to a polish filtration. Prior to the polish filtration vats and piping, contacting the filtrated product, were steamed. The filtrated product was then packed in 20 liter sealed drums.

**Table 3. Formulations of Rhizomucor miehei aspartic protease.**

| Ex | pH | NaCl (g/l) | Sodium acetate (g/l) | Sodium benzoate (g/l) | Initial enzyme activity (IMCU/ml) |
|---|---|---|---|---|---|
| 10 | 5.25 | 149 | 31 | - | 762 |
| 11 | 5.20 | 149 | 31 | - | 228 |

### Stability test

Samples were stored in closed drums of 20 1 in incubators set at 5°C or 20°C. One drum of formulation 10 and three drums of formulation 11 were used in this stability test. Microbial stability of these samples was checked at regular time intervals up to 8 months by the standard plate count. In all containers, at both 5 and 20°C no growth could be detected of bacteria, yeasts or moulds, as determined by the methods for standard plate count or yeasts and moulds count as described in Examples 1-5.

The microbial stability of these formulations without benzoate was good throughout the storage period of 8 months.

### Challenge test

Of each formulation a sample (50 ml) was inoculated with a mix of xerotolerant microorganisms (a halophilic *Micrococcus, Torulopsis candida* and *Hansenula anomala)* at approximately 2E+3 colony forming units per ml. These samples were stored in a bottle with screw top in an incubator set at 20°C. Both formulations showed a good resistance against against microorganisms.

Both the stability and challenge test demonstrate that benzoate free formulations, which contain acetate, have efficient preservative effect.

## Claims

1. Liquid composition comprising:
(i) a *Rhizomucor miehei* aspartic protease; and
(ii) an inorganic salt; and
(iii) a compound selected from formate, acetate, lactate, propionate, malate or fumarate
in which composition;
the sum concentration of sorbate, benzoate and alkyl esters of para-hydroxybenzoate is less than 0.010 mol/l;
the standard plate count ≤ 100 in 1 ml;
yeast count ≤10 in 1 ml; and
mould count ≤10 in 1 ml,
and wherein the pH is between 4.8 and 5.5; and
in which the concentration of formate, acetate, lactate, propionate, malate, or fumarate or combination thereof is at least 0.1 mol/l.

2. Composition according to claim 1, in which the concentration of formate, acetate, lactate, propionate, malate, or fumarate or combination thereof is at least 0.2 mol/l.

3. Composition according to claim 1, which comprises acetate.

4. Liquid composition according to any preceding claim, obtainable by the process according to any one of claims 16 to 18.

5. Composition according to any preceding claim, in which the concentration of benzoate is less than 0.010 mol/l.

6. Composition according to any preceding claim, in which the sum concentration of sorbate, benzoate and alkyl esters of para-hydroxybenzoate is less than 0.010 mol/l.

7. Composition according to any preceding claim, wherein said inorganic salt is NaCl, KCI, Na₂SO₄ or (NH₄)₂SO₄.

8. Composition according to any preceding claim, which comprises at least 80 g/l of an inorganic salt or of a combination of inorganic salts.

9. Composition according to any preceding claim, which comprises at least 80 g/l of NaCl.

10. Composition according to any preceding claim, in which the concentration of inorganic salt is less than 180 g/l.

11. Composition according to any preceding claim, which comprises less than 180 g/l of NaCl.

12. Composition according to any preceding claim, wherein the composition further comprises methionine.

13. Composition according to any preceding claim, wherein the standard plate count remains ≤ 100 in 1 ml, the yeast count remains ≤ 10 in 1 ml and mould count remain ≤ 10 in 1 ml during a period of at least 6 months, when the composition is stored in a closed container at a temperature of 4 °C in the dark.

14. Composition according to any preceding claim, wherein the enzymatic activity decreases at most 5% during a period of at least 6 months, when the composition is stored in a closed container at a temperature of 4 °C in the dark.

15. Closed container comprising the composition according to any preceding claim.

16. Process for preparing a liquid composition comprising a *Rhizomucor miehei* aspartic protease, said process comprising:
(a) providing a fermentation broth, said fermentation broth containing (i) micro-organisms that have produced the protease and (ii) supernatant containing the protease;
(b) separating, by solid liquid separation, supernatant from the fermentation broth;
(c) purifying the separated supernatant, to obtain a purified solution;
(d) adding one or more additives to the purified solution, wherein at least one of said one or more additives is an inorganic salt, a polyalcohol, or a compound selected from formate, acetate, lactate, propionate, malate, or fumarate; and
(e) filtering the purified solution containing said one or more additives, and
wherein the concentration of formate, acetate, lactate, propionate, malate, or fumarate or combination thereof is at least 0.1 mol/l.

17. Process according to claim 16, wherein no compound selected from benzoate, sorbate or alkyl ester of para-hydroxybenzoate is added to the purified solution.

18. Process according to any one of claims 16 to 17, wherein the sum concentration of sorbate, benzoate and alkyl esters of para-hydroxybenzoate in the filtered solution resulting from (e) is less than 0.010 mol/l.

19. Use of a composition according to any one of claims 1 to 14, as a coagulant in the production of cheese.

20. Process for preparing cheese, comprising, (i) supplementing milk with a composition according to any one of claims 1 to 14, to effect coagulation of the milk, wherein a curd is obtained; and (ii) processing the curd into cheese.

## Patentansprüche

1. Flüssige Zusammensetzung, umfassend:
(i) eine *Rhizomucor-miehei*-Aspartylprotease und
(ii) ein anorganisches Salz und
(iii) eine aus Formiat, Acetat, Lactat, Propionat, Malat oder Fumarat ausgewählte Verbindung;
wobei in der Zusammensetzung:
die Gesamtkonzentration von Sorbat, Benzoat und Alkylestern von para-Hydroxybenzoat weniger als 0,010 mol/l beträgt;
die Standardkeimzahl ≤ 100 in 1 ml ist;
die Hefekeimzahl ≤ 10 in 1 ml ist und
die Schimmelpilzkeimzahl ≤ 10 in 1 ml ist;
und wobei der pH-Wert zwischen 4,8 und 5,5 liegt; und
wobei die Konzentration von Formiat, Acetat, Lactat, Propionat, Malat oder Fumarat oder einer Kombination davon mindestens 0,1 mol/l beträgt.

2. Zusammensetzung nach Anspruch 1, wobei die Konzentration von Formiat, Acetat, Lactat, Propionat, Malat oder Fumarat oder einer Kombination davon mindestens 0,2 mol/l beträgt.

3. Zusammensetzung nach Anspruch 1, die Acetat umfasst.

4. Flüssige Zusammensetzung nach einem der vorhergehenden Ansprüche, die durch das Verfahren nach einem der Ansprüche 16 bis 18 erhältlich ist.

5. Zusammensetzung nach einem der vorhergehenden Ansprüche, wobei die Benzoatkonzentration weniger als 0,010 mol/l beträgt.

6. Zusammensetzung nach einem der vorhergehenden Ansprüche, wobei die Gesamtkonzentration von Sorbat, Benzoat und Alkylestern von para-Hydroxybenzoat weniger als 0,010 mol/l beträgt.

7. Zusammensetzung nach einem der vorhergehenden Ansprüche, wobei es sich bei dem anorganischen Salz um NaCl, KCl, Na₂SO₄ oder (NH₄)₂SO₄ handelt.

8. Zusammensetzung nach einem der vorhergehenden Ansprüche, die mindestens 80 g/l eines anorganischen Salzes oder einer Kombination von anorganischen Salzen umfasst.

9. Zusammensetzung nach einem der vorhergehenden Ansprüche, die mindestens 80 g/l NaCl umfasst.

10. Zusammensetzung nach einem der vorhergehenden Ansprüche, wobei die Konzentration an anorganischem Salz weniger als 180 g/l beträgt.

11. Zusammensetzung nach einem der vorhergehenden Ansprüche, die weniger als 180 g/l NaCl umfasst.

12. Zusammensetzung nach einem der vorhergehenden Ansprüche, wobei die Zusammensetzung ferner Methionin umfasst.

13. Zusammensetzung nach einem der vorhergehenden Ansprüche, wobei bei Lagerung der Zusammensetzung in einem geschlossenen Behälter bei einer Temperatur von 4°C im Dunkeln die Standardkeimzahl über einen Zeitraum von mindestens 6 Monaten ≤ 100 in 1 ml bleibt, die Hefekeimzahl über einen Zeitraum von mindestens 6 Monaten ≤ 10 in 1 ml bleibt und die Schimmelpilzkeimzahl über einen Zeitraum von mindestens 6 Monaten ≤ 10 in 1 ml bleibt.

14. Zusammensetzung nach einem der vorhergehenden Ansprüche, wobei bei Lagerung der Zusammensetzung in einem geschlossenen Behälter bei einer Temperatur von 4°C im Dunkeln die enzymatische Aktivität über einen Zeitraum von mindestens 6 Monaten um höchstens 5% abnimmt.

15. Geschlossener Behälter, der die Zusammensetzung nach einem der vorhergehenden Ansprüche umfasst.

16. Verfahren zur Herstellung einer eine *Rhizomucor-miehei*-Aspartylprotease umfassenden flüssigen Zusammensetzung, bei dem man:
(a) eine Fermentationsbrühe bereitstellt, wobei die Fermentationsbrühe (i) Mikroorganismen, die die Protease produziert haben, und (ii) Überstand, der die Protease enthält, enthält;
(b) durch Fest-Flüssig-Trennung den Überstand von der Fermentationsbrühe trennt;
(c) den abgetrennten Überstand reinigt, wobei man eine gereinigte Lösung erhält;
(d) ein oder mehrere Additive zu der gereinigten Lösung gibt, wobei es sich bei mindestens einem des einen bzw. der mehreren Additive um ein anorganisches Salz, einen Polyalkohol oder eine aus Formiat, Acetat, Lactat, Propionat, Malat oder Fumarat ausgewählte Verbindung handelt; und
(e) die das eine bzw. die mehreren Additive enthaltende gereinigte Lösung filtriert, und wobei die Konzentration von Formiat, Acetat, Lactat, Propionat, Malat oder Fumarat oder einer Kombination davon mindestens 0,1 mol/l beträgt.

17. Verfahren nach Anspruch 16, bei dem man keine aus Sorbat, Benzoat oder Alkylester von para-Hydroxybenzoat ausgewählte Verbindung zu der gereinigten Lösung gibt.

18. Verfahren nach einem der Ansprüche 16 bis 17, bei dem die Gesamtkonzentration von Sorbat, Benzoat und Alkylestern von para-Hydroxybenzoat in der filtrierten Lösung aus (e) weniger als 0,010 mol/l beträgt.

19. Verwendung einer Zusammensetzung nach einem der Ansprüche 1 bis 14 als Gerinnungsmittel bei der Herstellung von Käse.

20. Verfahren zur Herstellung von Käse, bei dem man (i) Milch mit einer Zusammensetzung nach einem der Ansprüche 1 bis 14 supplementiert, um die Milch zum Gerinnen zu bringen, wobei man einen Bruch erhält; und (ii) den Bruch zu Käse verarbeitet.

## Revendications

1. Composition liquide, comprenant :
(i) une protéase aspartique issue de *Rhizomucor miehei ;* et
(ii) un sel inorganique ; et
(iii) un composé choisi parmi formiate, acétate, lactate, propionate, malate ou fumarate ;
dans laquelle composition
la somme des concentrations en sorbate, benzoate et alkylesters de para-hydroxybenzoate est inférieure à 0,010 mol/l ;
la numération sur plaque standard est ≤ 100 dans 1 ml ;
la numération de levure est ≤ 10 dans 1 ml ; et
la numération de moisissures est ≤ 10 dans 1 ml ;
et dans laquelle le pH est compris entre 4,8 et 5,5 ; et
dans laquelle la concentration en formiate, acétate, lactate, propionate, malate ou fumarate, ou en une combinaison de ceux-ci, est d'au moins 0,1 mol/l.

2. Composition selon la revendication 1, dans laquelle la concentration en formiate, acétate, lactate, propionate, malate ou fumarate, ou en une combinaison de ceux-ci, est d'au moins 0,2 mol/l.

3. Composition selon la revendication 1, qui comprend de l'acétate.

4. Composition liquide selon l'une quelconque des revendications précédentes, pouvant être obtenue par le procédé selon l'une quelconque des revendications 16 à 18.

5. Composition selon l'une quelconque des revendications précédentes, dans laquelle la concentration en benzoate est inférieure à 0,010 mol/l.

6. Composition selon l'une quelconque des revendications précédentes, dans laquelle la somme des concentrations en sorbate, benzoate et alkylesters de para-hydroxybenzoate est inférieure à 0,010 mol/l.

7. Composition selon l'une quelconque des revendications précédentes, dans laquelle ledit sel inorganique est NaCl, KCl, Na₂SO₄ ou (NH₄)₂SO₄.

8. Composition selon l'une quelconque des revendications précédentes, qui comprend au moins 80 g/l d'un sel inorganique ou d'une combinaison de sels inorganiques.

9. Composition selon l'une quelconque des revendications précédentes, qui comprend au moins 80 g/l de NaCl.

10. Composition selon l'une quelconque des revendications précédentes, dans laquelle la concentration en sel inorganique est inférieure à 180 g/l.

11. Composition selon l'une quelconque des revendications précédentes, qui comprend moins de 180 g/l de NaCl.

12. Composition selon l'une quelconque des revendications précédentes, **caractérisée en ce que** la composition comprend en outre de la méthionine.

13. Composition selon l'une quelconque des revendications précédentes, dans laquelle la numération sur plaque standard reste ≤ 100 dans 1 ml, la numération de levure reste ≤ 10 dans 1 ml, et la numération de moisissures reste ≤ 10 dans 1 ml, pendant une période d'au moins 6 mois, lorsque la composition est stockée dans un conteneur fermé à une température de 4°C à l'obscurité.

14. Composition selon l'une quelconque des revendications précédentes, dans laquelle l'activité enzymatique diminue d'au plus 5% pendant une période d'au moins 6 mois, lorsque la composition est stockée dans un conteneur fermé à une température de 4°C à l'obscurité.

15. Conteneur fermé comprenant la composition selon l'une quelconque des revendications précédentes.

16. Procédé de préparation d'une composition liquide comprenant une protéase aspartique issue de *Rhizomucor miehei,* ledit procédé comprenant :
(a) la fourniture d'un bouillon de fermentation, ledit bouillon de fermentation contenant (i) des microorganismes ayant produit la protéase et (ii) un surnageant contenant la protéase ;
(b) la séparation, par séparation solide-liquide, du surnageant à partir du bouillon de fermentation ;
(c) la purification du surnageant séparé afin d'obtenir une solution purifiée ;
(d) l'addition d'un ou plusieurs additifs à la solution purifiée, où au moins l'un parmi lesdits un ou plusieurs additifs est un sel inorganique, un polyalcool, ou un composé choisi parmi formiate, acétate, lactate, propionate, malate ou fumarate ; et
(e) la filtration de la solution purifiée contenant ledit un ou plusieurs additifs ; et
où la concentration en formiate, acétate, lactate, propionate, malate ou fumarate, ou en une combinaison de ceux-ci, est d'au moins 0,1 mol/l.

17. Procédé selon la revendication 16, dans lequel aucun composé choisi parmi benzoate, sorbate et alkylester de para-hydroxybenzoate, n'est ajouté à la solution purifiée.

18. Procédé selon l'une quelconque des revendications 16 à 17, dans lequel la somme des concentrations en sorbate, benzoate et alkylesters de para-hydroxybenzoate dans la solution filtrée résultant de (e) est inférieure à 0,010 mol/l.

19. Utilisation d'une composition selon l'une quelconque des revendications 1 à 14, comme agent coagulant dans la production de fromage.

20. Procédé de préparation de fromage, comprenant (i) la complémentation de lait par une composition selon l'une quelconque des revendications 1 à 14, afin d'obtenir une coagulation du lait, où on obtient un caillé ; et (ii) le traitement du caillé en fromage.
